# EUROPÄISCHE PATENTSCHRIFT

(11) **EP 1 985 269 B1**
(45) Veröffentlichungstag und Bekanntmachung des Hinweises auf die Patenterteilung: **27.07.2011**
(21) Anmeldenummer: 07008446.2
(22) Anmeldetag: 25.04.2007
(51) Int. Cl.: A61F 9/01

(54) **Vorrichtung, Verfahren und Steuerprogramm für die refraktive Chirurgie**
Device, method and control program for refractive surgery
Dispositif, procédé et programme de commande pour la chirurgie réfractive

(43) Veröffentlichungstag der Anmeldung: 29.10.2008
(73) Patentinhaber: WaveLight GmbH, 91058 Erlangen (DE)
(72) Erfinder: Lemonis, Sissimos, 90571 Schwaig (DE)
(74) Vertreter: von Hellfeld, Axel

(56) Entgegenhaltungen:
- EP-B1- 1 316 287
- WO-A-03/011177
- US-A1- 2004 019 346
- US-A1- 2005 137 586

## Beschreibung

Die Erfindung betrifft eine Vorrichtung für die refraktive Chirurgie, ein Steuerprogramm für eine solche Vorrichtung und ein Verfahren zum Erzeugen eines solchen Steuerprogramms.

Unter der "refraktiven Chirurgie" verstehen Fachkreise die Änderung der Abbildungseigenschaften des optischen Systems "Auge" durch Laserstrahlung. Die Laserstrahlung ändert also die brechenden Eigenschaften einer order mehrerer Komponenten des Auges. Da für die Abbildungseigenschaft des Auges hauptsächlich die Kornea maßgeblich ist, wird mit der refraktiven Chirurgie insbesondere eine Formung der Kornea durchgeführt.

Prominentes Beispiel einer solchen Neuformung der Kornea zur Änderung von deren refraktiven Eigenschaften ist die LASIK. Die vorliegende Erfindung betrifft insbesondre die LASIK-Technik. Darüber hinaus ist die Erfindung auch einsetzbar allgemein in der PRK und in der EPI-LASIK. Auch bei Einsatz von Femtosekundenlasern kann die Erfindung zum Einsatz kommen.

Bei der LASIK wird gemäß dem Stand der Technik ein sogenanntes Ablationsprofil bestimmt, d.h. es wird aufgrund von Messungen am Auge und gegebenenfalls anderen Einflussgrößen berechnet, an welcher Stelle der Kornea wie viel Gewebe (Stroma) abzutragen ist, damit nach dem Abtrag die Kornea eine für das zu behandelnde Auge optimale Form hat, d.h. die zuvor vorhandenen optischen Abbildungsfehler des Auges soweit als möglich korrigiert sind.

Für die genannte Berechnung des Ablationsprofils kennt der Stand der Technik vielfältige Verfahren.

Ist für das zu behandelnde Auge das Ablationsprofil bestimmt, dann wird berechnet, wie dieses Profil mit Laserstrahlung am besten von der Kornea abgetragen (ablatiert) werden kann. Hierzu wird eine Folge einzelner Laserpulse in Raum und Zeit berechnet, die in Wechselwirkung mit dem Stroma die gewünschte Neuformung der Kornea bewirkt.

Das Ablationsprofil ist eine drei-dimensionale Raumform und entspricht einem Volumen der Kornea, das entfernt werden soll.

Die Mittel, Laserstrahlung mit einem Rechner so zu steuern, dass ein vorgegebenes Ablationsprofil abgetragen wird, sind als solches im Stand der Technik gut bekannt.

Bei der Durchführung der refraktiven Chirurgie steuert der Rechner gemäß einem Steuerprogramm die Laserstrahlung, zum Beispiel einzelne Laserpulse ("Spots") in einer Raum- und Zeitfolge über das Auge.

Bei dieser Steuerung der Laserstrahlung in Bezug auf das Auge ist eine ganz entscheidende Bezugsgröße das sogenannte Ablationszentrum. Das Ablationszentrum ist der räumliche Bezugspunkt (Referenzpunkt), auf den die genannte Raumfolge der Laserpulse bezogen ist. Im Stand der Technik wird üblicherweise der Mittelpunkt (das Zentrum) der Pupille als Ablationszentrum herangezogen. Die Pupille, also die von der Iris als Blende freigelassene Öffnung für den Durchtritt von Strahlung in das Auge, hat eine relativ scharfe Kontur und lässt sich deshalb gut mit einer Kamera aufnehmen und mit Bildverarbeitungsprogrammen analysieren. Solche Aufnahmeeinrichtungen und Verarbeitungsprogramme sind als solche im Stand der Technik gut bekannt und darauf kann die vorliegende Erfindung teilweise zurückgreifen.

Während der refraktiven Chirurgie ist das zu behandelnde Auge keine konstante Größe, vielmehr können sich während des Eingriffs Eigenschaften und auch die Orientierung des Auges ändern. Änderungen der Orientierung des Auges werden gemäß dem Stand der Technik mit einem sogenannten Eye-Tracker verfolgt. Ein Eye-Tracker verfolgt Bewegungen des Auges üblicherweise durch die bereits erwähnte Aufnahme der Pupille mit einer Kamera und mit anschließender Bildverarbeitung. Bewegungen des Auges macht die Pupille mit und somit können auf diese Weisen die Bewegungen festgestellt werden und es kann die Steuerung des Laserstrahls solchen Augenbewegungen nachgeführt werden, d.h. das zuvor berechnete Ablationsprofil wird trotz Augenbewegungen während der Operation, die in der Regeln nicht zuverlässig ausgeschlossen werden können, genau abgetragen.

Wie oben erwähnt, verwendet der Stand der Technik in der Regel das Zentrum der Pupille als Ablationszentrum. Die vorliegende Erfindung will diesen Stand der Technik verbessern und geht dabei von folgenden Überlegungen aus:

Während der refraktiven Chirurgie hat auch die Pupille in aller Regel keine gleichbleibende Größe und Form. Die Pupille hat auch in der Regel keine perfekte Kreisform, vielmehr hat sie regelmäßig Abmessungen, die in einer Richtung länger sind als in einer anderen Richtung. Die Abmessungen der Pupille hängen bekanntlich von der einfallenden Lichtmenge ab und das Auge adaptiert den Pupillenquerschnitt als Funktion der einfallenden Lichtmenge. Mit einer Änderung der Größe der Pupille geht aber in aller Regel auch eine Änderung der Verlagerung des Pupillenzentrums einher. Mit anderen Worten, weitet sich die Pupille auf, dann ist diese Aufweitung nicht konzentrisch und das Pupillenzentrum verschiebt sich dabei in aller Regel (unter dem Pupillenzentrum kann man bei nicht kreisförmiger Gestalt zum Beispiel den Flächenschwerpunkt verstehen). Komplexer werden die Verhältnisse noch dadurch, dass mit einer Flächenänderung der Pupille in aller Regel auch eine Drehung der nicht kreisförmigen Pupillenform einher geht, was in der Anatomie als Zyklotorsion bekannt ist.

Wenn im Stand der Technik das Pupillenzentrum als Ablationszentrum während der refraktiven Chirurgie herangezogen wird, ergeben sich deshalb eine Reihe von systematischen Fehlerquellen.

Zunächst gilt, dass das Pupillenzentrum nicht exakt auf der optischen Achse des Auges liegt. Es werden mit Blick auf das optische System "Auge" unterschiedliche Achsen definiert, für die vorliegende Erfindung sind insbesondere von Bedeutung die optische Achse und die visuelle Achse (Visualachse). Die optische Achse verbindet gemäß einer üblichen Definition die Krümmungsmittelpunkte der brechenden Oberflächen der Augenkomponenten, d.h. sie steht in der Regel senkrecht auf allen brechenden Flächen. Die Visusachse ist üblicherweise die Linie, welche den vom Auge fixierten Punkt und die Fovea verbindet. Diese Linie geht in der Regel durch den sogenannten Nodalpunkt an der rückwärtigen Oberfläche der Linse, und zwar an einer Stelle, an der auch die optische Achse diesen Punkt in der Regel durchläuft.

Die optische Achse geht also in der Regel nicht durch die Fovea. Der Winkel zwischen der optischen Achse und der Visusachse ist typischerweise im Bereich von 5°.

Die Erfindung geht aus von der Erkenntnis, dass verbesserte Ergebnisse der refraktiven Chirurgie dann erreicht werden können, wenn als Referenzpunkt für die Ablation, also als sogenanntes Ablationszentrum, weder ein Zentrum der Pupille herangezogen wird, noch ein Punkt auf der optischen Achse. Bei Verwendung des Pupillenzentrums als Ablationszentrum ergibt allein schon die oben genannte Verschiebung des Pupillenzentrums in Abhängigkeit von der Pupillengröße regelmäßig einen systematischen Fehler und nur zufällig kann bei bestimmten vorteilhaften Eigenschaften des gerade behandelten Auges eine Zentrierung der Ablation auf das Pupillenzentrum zu guten Ablationsergebnissen führen.

Auch wenn die als solches anatomisch bekannte Verschiebung des Pupillenzentrums bei Veränderung der Größe der Pupille bei der Bestimmung des Ablationszentrums berücksichtig würde, wäre damit nicht regelmäßig eine Verbesserung der Ablation verbunden, weil der mit dieser Auswahl verbundene systematische Fehler quasi mitgeschleppt würde.

Die vorstehenden Erörterungen gelten auch für den Fall, dass zur Berechnung des Ablationsprofils und zur Generierung des Steuerprogrammes für die Laserstrahlung das Auge mit einer Wellenfrontanalyse vermessen wird, zum Beispiel gemäß Hartmann-Shack oder Tscherning oder durch Topographiemessungen. Auch bei sogenannten "Standard"-Ablationen bedarf es einer Bestimmung des Albationsprofils mit hoher Genauigkeit.

Die WO 03/011177 A2 lehrt die Zentrierung einer Ablation auf die Visusachse in Bezug auf das Pupillenzentrum.

Die US 2004/019346 A1 beschreibt ein Verfahren zur Ablation an der Kornea, bei dem die Laserstrahlung entsprechend den Winkeln zwischen der Korneaoberfläche und dem Laserstrahl gesteuert wird. Der jeweilige örtliche Einfallswinkel der Strahlung wird herangezogen, um die örtlich erforderliche Ablation des Gewebes zu bestimmen. Eine so ermittelte Ablationskarte (Ablationsprofil) verwendet den Apex der Korneaoberfläche nicht zur Ausrichtung des Ablationsprofils auf den Apex, insbesondere nicht unter Verwendung der Abhängigkeit der Lage des Apex von einer Eigenschaft der Pupille.

Die EP1923027 beschreibt eine Vorrichtung, die die Lage des Apex bestimmt und die Ablation steuert aufgrund von einer geometrischen Funktion für ein quasi ideales Auge.

Der Erfindung liegt die Aufgabe zugrunde, für die refraktive Chirurgie ein mit relativ einfachen Mitteln zu bestimmendes Ablationszentrum anzugeben, mit dem verbesserte refraktive Ergebnisse erreichbar sind.

Hierzu lehrt die Erfindung eine Vorrichtung für die refraktive Chirurgie mit den Merkmalen des Patentanspruchs 1. Weiterhin lehrt die Erfindung ein Steuerprogramm für eine solche Vorrichtung mit den Merkmalen des Patentanspruchs 5.

Bevorzugte Ausgestaltungen sind in den abhängigen Ansprüchen beschrieben.

Der Erfindung liegt also die Erkenntnis zugrunde, dass verbesserte Ablationsergebnisse dann erreicht werden können, wenn während des chirurgischen Eingriffs die Ablation auf den Apex der Kornea zentriert wird, der auf der Vorderfläche der Kornea liegt.

Der Apex ist der höchste Punkt der Kornea, ihr Scheitelpunkt.

Nun sind die vorstehend genannten Punkte auf der Oberfläche der Kornea während des chirurgischen Eingriffs in aller Regel nicht einfach zu bestimmen, jedenfalls nicht mit bekannten, zur Verfügung stehenden Mitteln.

Die Erfindung lehrt deshalb, für das zu behandelnde Auge empirisch die funktionale Abhängigkeit der räumlichen Lage des vorstehend genannten Punktes auf der Korneaoberfläche (der als Ablationszentrum dienen soll) von der Form und Lage der Pupille zu bestimmen. Wie oben ausgeführt ist, wird die Pupille bei refkrativen Eingriffen sowieso in aller Regel mit einer Kamera und mit hinreichender Frequenz vermessen, so dass die Laserstrahlführung Bewegungen des Auges mit macht (Eye-Tracker). Wird nun erfindungsgemäß mit einer Kamera und als solches sogar weitgehend bekannten Bildverarbeitungsprogrammen die Pupille vermessen und für das zu behandelnde Auge die Abhängigkeit der Lage zum Beispiel des Apex als Funktion der Pupillendaten gemessen und werden diese Messergebnisse in dem Rechner, der später die refraktive Chirurgie, also im Wesentlichen den Laserstrahl, steuert abgelegt, dann kann der steuernde Rechner den Laserstrahl, d.h. das Ablationsprofil auf den Apex als Referenzpunkt (Ablationszentrum) beziehen. Dazu muss dann nur während des refraktiven Eingriffs die Pupille bzw. die Iris vermessen werden, was, wiegesagt, aus anderen Gründen sowieso erfolgt.

Als zu vermessende Eigenschaften der Pupille, welche den genannten funktionalen Zusammenhang zwischen dem ausgewählten Ablationszentrum auf der Kornea und während der Operation messbaren Größen darstellt, kommen insbesondere in betracht bestimmte Abmessungen der Pupille und sich ihre daraus ergebende Form. Eine weitere Verbesserung ist dadurch möglich, dass diese funktionale Abhängigkeit zwischen Ablationszentrum und Messgrößen auch Eigenschaften der Iris berücksichtigt. Wie oben ausgeführt ist, dreht sich die Iris üblicherweise bei Veränderung der Pupillengröße und diese Drehung kann dadurch erkannt werden, dass sich bestimmte Strukturen der Iris drehen, was durch Bildverarbeitung des mit der Kamera aufgenommenen Bildes erkennbar ist.

Die genannte funktionale Abhängigkeit zwischen der Lage z.B. des Apex und Eigenschaften der Pupille und/oder der Iris kann zum Beispiel in der Art einer Speichertabelle im Rechner abgelegt werden.

Die Messung der funktionalen Abhängigkeit der Position des Apex (oder eines anderen erfindungsgemäßen ausgewählten Ablationszentrums) von Messdaten der Pupille, wie insbesondere deren Größe und Lage, kann zum Beispiel so erfolgen, dass (vor der refraktiven Chirurgie, versteht sich) beim zu behandelnden Auge der Apex mit geeigneten Mitteln bestimmt wird, zum Beispiel mit einem Topometer. Dann kann diese Position des Apex mit Markierungen auf der Kornea festgehalten werden, die vorzugsweise nicht direkt auf dem Apex liegen. Zum Beispiel können sogenannte Keratometermarken nahe dem Apex angebracht werden, die die räumliche Lage des Apex definieren, d.h. der bildverarbeitende Rechner erkennt aus den Markierungen die Lage des Apex. Sodann wird der genannte funktionale Zusammenhang zwischen den Eigenschaften der Pupille/Iris und der Apex-Position bestimmt (wiederum vor dem eigentlichen chirurgischen Eingriff).

Die Erfindung stellt auch bereit, zum Beispiel auf einem Datenträger, ein Steuerprogramm für eine Vorrichtung für die refraktive Chirurgie, welche folgendes aufweist:
- eine Laserstrahlquelle,
- Mittel zum Formen und Führen des von der Laserstrahlquelle emittierten Laserstrahls in bezug auf ein zu behandelndes Auge,
- eine Kamera zum Aufnehmen der Iris und Pupille des Auges,
- einen Rechner der das Steuerprogramm zum Steuern der genannten Mittel entsprechend einem Ablationsprofil ausführt, wobei
   das Steuerprogramm:
- eine Funktion der Lage eines vorgegebenen Punktes der Kornea des Auges in Abhängigkeit von zumindest einer Eigenschaft der Pupille enthält,
- währen der refraktiven Chirurgie die momentane Eigenschaft der Pupille mit der Kamera aufnimmt und daraus mittels der genannten Funktion die Lage des vorgegebenen Punktes der Kornea bestimmt, und
das Ablationsprofil auf die so bestimmte Lage des Punktes ausrichtet.

Auch bei diesem Steuerprogramm können im Einzelnen die oben zu der Vorrichtung beschriebenen Ausgestaltungen, insbesondere hinsichtlich der Auswahl des Ablationszentrums und der für die Messungen herangezogenen Eigenschaften der Pupille eingesetzt werden.

Die Erfindung betrifft auch ein Verfahren zum Erzeugen eines Steuerprogramms für die refraktive Chirurgie, mit dem Laserstrahlung gemäß einer vorgegebenen Raum- und Zeitfolge auf oder in ein zu behandelndes Auge gerichtet wird, wobei die vorgegebene Raum- und Zeitfolge in bezug auf einen Ort des Auges ausgerichtet wird, und wobei
- vor der refraktiven Chirurgie beim zu behandelnden Auge die Abhängigkeit der Position des genannten Ortes von einer Eigenschaft der Pupille; die sich während der refraktiven Chirurgie ändern kann, bestimmt und abgespeichert wird, wobei der Ort auf oder in der Kornea des Auges liegt.

Der vorstehend genannte Ort des Auges ist insbesondere das oben genannte Ablationszentrum, der Apex der Kornea. Auch können bei dem erfindungemäßen Verfahren zum Erzeugen des Steuerprogrammes die oben genannten Parameter bezüglich der Pupilleneigenschaften herangezogen werden.

Nachfolgend werden Ausführungsbeispiele der Erfindung anhand der Zeichnungen näher erläutert. Es zeigt:
- Fig. 1: schematisch einen Schnitt durch ein Auge mit den hier besonders interessie- renden Achsen und Schnittpunkten; und
- Fig.2: schematisch eine Vorrichtung für die refraktive Chirurgie.

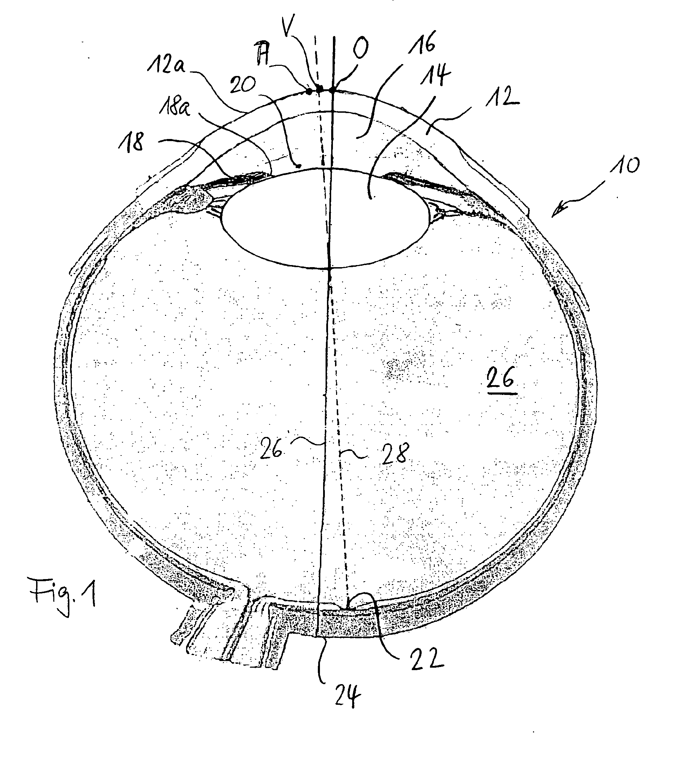

Der Saggitalschnitt durch ein Auge 10 gemäß Fig.1 zeigt schematisch eine Kornea 12, eine Linse 14, eine Vorderkammer 16, eine Iris 18, deren Rand 18a eine Pupille 20 begrenzt, eine Fovea 22, eine Macula Lutea 24, eine optische Achse 26 und eine Visusachse 28.

Die optische Achse 26 schneidet die Vorderfläche 12a der Kornea 12 an der Stelle O. Die Visusachse 28 schneidet die Vorderfläche 12a der Kornea an der Stelle V.

Der Apex A der Kornea 12 liegt regelmäßig weder an der Stelle O noch an der Stelle V sondern, wie in Fig.1 schematisch gezeigt, so, dass der Schnittpunkt V der Visusachse (visuellen Achse) zwischen Apex A und dem Schnittpunkt O der optischen Achse mit der Korneaoberfläche liegt. Dabei liegt V in der Regel näher an A als an O. Diese anatomischen Regelmäßigkeiten macht sich die Erfindung zu Nutze.

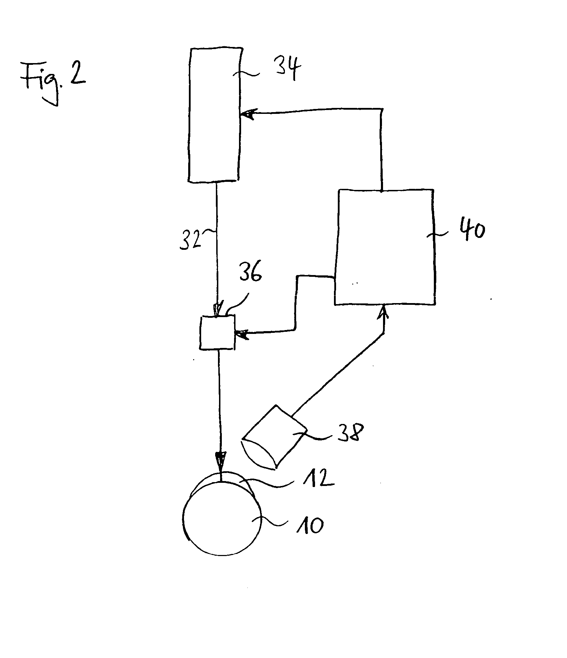

Fig.2 zeigt schematisch eine Vorrichtung für refraktive Chirurgie mit einer Laserstrahlquelle 34, die einen Laserstrahl 32 emittiert, der mit Einrichtungen 36 zur Strahlformung und -führung auf ein zu behandelndes Auge 10 gerichtet wird. Mit einer Kamera, zum Beispiel einer IR-Kamera 38 wird insbesondere die Pupille und die Iris des Auges aufgenommen und das digitale Bildsignal wird in einen Rechner 40 eingegeben.

Der Rechner 40 steuert alle genannten Komponenten, also insbesondere die Laserstrahlquelle 34 und die Mittel 36 zur Strahlformung und -führung. Die bisher beschriebenen Komponenten sind als solches im Stand der Technik gut bekannt und werden hier nicht näher erläutert.

Für ein zu behandelndes Auge wird zunächst in an sich bekannter Weise das oben erläuterte Ablationsprofil bestimmt. Dies geschieht durch Vermessung des Auges, z.B. durch Wellenfronanalyse. Das so gewonnene Ablationsprofil wird im Rechner 40 abgelegt. Der Rechner 40 enthält weiterhin ein Steuerprogramm zum Steuern von insbesondere den Einrichtungen 36 zur Strahlformung und -führung gemäß dem Ablationsprofil. Dies ist als solches ebenfalls im Stand der Technik gut bekannt.

Zunächst wird der Apex A der Kornea des zu behandelnden Auges bestimmt, d.h. die Raumkoordinaten des Apexpunktes A werden gemessen. Dies kann z.B. mittels sogenannter Keratometermarken durchgeführt werden, d.h. auf der Oberfläche 12a der Kornea werden nahe dem Apex A eine oder mehrere Markierungen gemacht, die die Position des Apex A definieren. Diese Vermessung des Apex kann z.B. mit einem herkömmlichen Topometer erfolgen. Solche Markierungen sind hernach auf den mit der Kamera 38 erzeugten Bildern erkennbar und die im Rechner 40 abgelegten Bildverarbeitungsprogramme "erkennen" aufgrund dieser Markierungen den Apex A. Die Koordinaten des Apex A können fortan als für das Auge konstant angenommen werden und dienen als Referenzpunkt, d.h. als Ablationszentrum für eine später erfolgende Ablation, wobei dieser Referenzpunkt auch bei sich ändernden Pupillenweiten und auch Pupillenrotation unveränderlich bleibt.

Nach der Bestimmung der Raumkoordinaten des Apex A wird in einem weiteren Schritt für das zu behandelnde Auge die funktionale Abhängigkeit der Position des Apex A von der individuellen Pupillenbewegung, also der Änderung der Pupillenweite und der Pupillenrotation, ermittelt.

Hierzu wird zunächst die Abhängigkeit der Lage des Pupillenzentrums von der Pupillenweite bestimmt. Wie oben erläutert ist, ist eine Aufweitung der Pupille in aller Regel nicht konzentrisch, d.h. nach einer Aufweitung der Pupille hat sich das Pupillenzentrum in der Regel verschoben. Diese Abhängigkeit der Lage des Pupillenzentrums von der Pupillenweite wird dadurch bestimmt, dass stufenweise unterschiedliche Pupillenweiten erzeugt werden und zu jeder Pupillenweite die Koordinaten des Pupillenzentrums mit dem Apex als Referenzpunkt bestimmt werden. Dabei erfolgt die Variation der Pupillenweite durch Änderung des eingestrahlten sichtbaren Lichtes, so dass die natürlich gegebene Pupillenweitung erfolgt (eine pharmakologische Weitung der Pupille weicht häufig von deren natürlicher Bewegung ab). Mittels einer Infrarotkamera wird dann für die verschiedenen Pupillenweiten jeweils die Lage des Pupillenzentrums ermittelt mit dem zuvor bestimmten Apex als Referenzpunkt.

Analog wird die Anhängigkeit des Winkels der Pupillenrotation von der Pupillenweite bestimmt. Dabei erkennt die IR-Kamera neben dem Pupillenzentrum auch die Veränderung der Lage des Iris-Musters, die Informationen über die Pupillendrehung liefert.

Auf diese Weise wird eine Funktion ermittelt, welche die Lage des Apex A in Abhängigkeit von momentan gegebenen Eigenschaften der Pupille darstellt, wie insbesondere der Pupillenweite und dem Drehzustand der Pupille. Diese empirisch gemessene Abhängigkeit für das zu behandelnde Auge wird im Rechner 40 abgespeichert und dann bei der hernach durchzuführenden refraktiven Chirurgie, also insbesondere LASIK, verwendet, um das momentane Ablationszentrum zu errechnen. Die Ablation wird also bei diesem Beispiel auf den Apex A zentriert.

Dies geschieht bei der refraktiven Chirurgie dadurch, dass mit einer Frequenz, die hinreichend hoch ist, um Bewegungen und Pupillenänderungen des Auges so schnell zu erfassen, dass die Ablation auf solche Änderungen eingestellt wird, die Pupille 20 und Iris 14 des Auges mit der IR-Kamera 38 aufgenommen wird. Die im Rechner 40 gespeicherten Bildverarbeitungsprogramme errechnen mit Hilfe der oben beschriebenen, abgespeicherten Funktionen zu der momentanen Pupillengröße und Pupillenlage die Koordinaten des Apex A und bei der Steuerung der Einrichtungen 36 für sie Strahlführung legt der Rechner 40 diesen momentanen Apex A als Ablationszentrum zugrunde, d.h. die einzelnen Laserpulse werden gemäß dem Ablationsprofil positioniert, bei dem der momentan gemessene Ort des Apex A als Ablationszentrum dient. Dies wird während des gesamten refraktiven chirurgischen Eingriffs mit der genannten hohen Frequenz immer wieder durchgeführt, so dass das momentane Ablationszentrum immer dem tatsächlichen Zustand des Auges entspricht. Parallel dazu wird während des Verfahrens mit der Kamera 38 und dem Rechner 40 auch das als solches bekannte "Eye-Tracking" durchgeführt.

Die vorstehend beschriebene Zentrierung der Ablation auf den Apex A bringt bereits eine Verbesserung des Ergebnisses der refraktiven Chirurgie, d.h. eine Verbesserung der Visuskorrektur.

## Patentansprüche

1. Vorrichtung für die refraktive Chirurgie mit
- einer Laserstrahlquelle (34),
- Mitteln (36) zum Formen und Führen des von der Laserstrahlquelle emittierten Laserstrahls in Bezug auf ein zu behandelndes Auge (10),
- einer Kamera (38) zum Aufnehmen der Iris und Pupille des Auges,
- einem Rechner (40) mit einem Steuerprogramm zum Steuern der genannten Mittel entsprechend einem Ablationsprofil,
**dadurch gekennzeichnet, dass**
das Steuerprogramm:
- eine für das zu behandelnde Auge empirisch gewonnene Funktion der Lage des Apex (A) der Kornea (12) des Auges (10) in Abhängigkeit von zumindest einer Eigenschaft der Pupille enthält,
- während der refraktiven Chirurgie die momentane Eigenschaft der Pupille mit der Kamera aufnimmt und daraus mittels der genannten Funktion die Lage des Apex (A) der Kornea bestimmt, und
- das Ablationsprofil auf die so bestimmte Lage des Apex (A) ausrichtet.

2. Vorrichtung nach Anspruch 1,
**dadurch gekennzeichnet, dass** die Eigenschaft der Pupille eine oder mehrere ihrer Abmessungen ist bzw. sind.

3. Vorrichtung nach einem der vorhergehenden Ansprüche,
**dadurch gekennzeichnet, dass** die Eigenschaft der Pupille ihre Form und/oder ihr Zentrum ist.

4. Vorrichtung nach einem der vorhergehenden Ansprüche,
**dadurch gekennzeichnet, dass** das Steuerprogramm auch mittels einer aufgenommenen Eigenschaft der Iris die momentane Lage des Apex der Kornea bestimmt.

5. Steuerprogramm für eine Vorrichtung für die refraktive Chirurgie, welche folgendes aufweist:
- eine Laserstrahlquelle (34),
- Mittel (36) zum Formen und Führen des von der Laserstrahlquelle emittierten Laserstrahls in bezug auf ein zu behandelndes Auge (10),
- eine Kamera (38) zum Aufnehmen der Iris und Pupille des Auges,
- einen Rechner (40) der das Steuerprogramm zum Steuern der genannten Mittel entsprechend einem Ablationsprofil ausführt,
**dadurch gekennzeichnet, dass** das Steuerprogramm:
- eine für das zu behandelnde Auge empirisch gewonnene Funktion der Lage des Apex (A) der Kornea (12) des Auges (10) in Abhängigkeit von zumindest einer Eigenschaft der Pupille enthält,
- währen der refraktiven Chirurgie die momentane Eigenschaft der Pupille mit der Kamera aufnimmt und daraus mittels der genannten Funktion die Lage des Apex (A) der Kornea bestimmt,
- das Ablationsprofil auf die so bestimmte Lage des Apex (A) ausrichtet.

6. Verfahren zum Erzeugen eines Steuerprogramms für die refraktive Chirurgie, mit dem Laserstrahlung gemäß einer vorgegebenen Raum- und Zeitfolge auf oder in ein zu behandelndes Auge gerichtet wird, wobei die vorgegebene Raum- und Zeitfolge in bezug auf einen Ort des Auges ausgerichtet wird, **dadurch gekennzeichnet, dass**
- vor der refraktiven Chirurgie beim zu behandelnden Auge die Abhängigkeit der Position des Apex (A) von einer Eigenschaft der Pupille, die sich während der refraktiven Chirurgie ändern kann, empirisch bestimmt und abgespeichert wird.

## Claims

1. Apparatus for refractive surgery comprising
- a laser beam source (34),
- means (36) for forming and guiding the laser beam emitted by the laser beam source with regard to an eye (10) to be treated,
- a camera (38) for taking pictures of the iris and the pupil of the eye,
- a computer (40) comprising a control program for controlling said means in accordance with an ablation profile ,
**characterized in that**
the control program:
- comprises, in connection with the eye to be treated, an empirically obtained function of the position of the apex (A) of the cornea (12) of the eye (10) in dependency from at least one property of the pupil,
- takes, during the refractive surgery, the actual property of the pupil by means of the camera and determines on the basis of said function the position of the apex (A) of the cornea, and
- adjusts the ablation profile to the such determined position of the apex (A).

2. Apparatus according to claim 1, **characterized in that** the property of the pupil is one or more of its dimensions.

3. Apparatus according to one of the preceding claims, **characterized in that** the property of the pupil is its shape and/or its center.

4. Apparatus according to one of the preceding claims, **characterized in that** the control program determines the actual position of the apex of the cornea also on the basis of a photographed property of the iris.

5. Control program for an apparatus for refractive surgery comprising:
- a laser beam source (34),
- means (36) for forming and guiding the laser beam emitted by the laser beam source with regard to the eye (10) to be treated,
- a camera (38) for taking pictures of the iris and the pupil of the eye,
- a computer (40) implementing the control program for controlling said means in accordance with an ablation profile,
**characterized in that** the control program:
- comprises, in connection with the eye to be treated, an empirically obtained function of the position of the apex (A) of the cornea (12) of the eye (10) in dependency from at least one property of the pupil,
- takes, during the refractive surgery, the actual property of the pupil by means of the camera and determines on the basis of said function the position of the apex (A) of the cornea, and
- adjusts the ablation profile to the such determined position of the apex (A).

6. Method for generating a control program for refractive surgery, said program being adapted to direct laser radiation in accordance with a pre-given sequence in space and time onto or into an eye to be treated, wherein the pre-given sequence in space and time is adjusted to a site of the eye,
**characterized in that**
- before the refractive surgery, with regard to the eye to be treated, the dependency of the position of the apex (A) from a property of the pupil, which may change during the refractive surgery, is determined empirically and stored.

## Revendications

1. Dispositif destiné à la chirurgie réfractive, comprenant
- une source de rayon laser (34),
- des moyens (36) pour former et guider le rayon laser émis par la source de rayon laser par rapport à un oeil à traiter (10),
- une caméra (38) pour enregistrer l'iris et la pupille de l'oeil,
- un ordinateur (40) avec un programme de commande pour commander lesdits moyens conformément à un profil d'ablation,
**caractérisé en ce que**
le programme de commande :
- comporte une fonction acquise empiriquement pour l'oeil à traiter et concernant la position du sommet (A) de la cornée (12) de l'oeil (10) en fonction d'au moins une propriété de la pupille,
- enregistre à l'aide de ladite caméra la propriété momentanée de la pupille pendant l'intervention réfractive et calcule à partir de cette propriété la position du sommet (A) de la cornée au moyen de ladite fonction et
- centre le profil d'ablation sur la position du sommet (A) ainsi déterminée.

2. Dispositif selon la revendication 1,
**caractérisé en ce que** la propriété de la pupille consiste en une ou plusieurs de ses dimensions.

3. Dispositif selon l'une des revendications précédentes,
**caractérisé en ce que** la propriété de la pupille consiste en sa forme et/ou en son centre.

4. Dispositif selon l'une des revendications précédentes,
**caractérisé en ce que** le programme de commande calcule également au moyen d'une propriété enregistrée de l'iris la position momentanée du sommet de la cornée.

5. Programme de commande pour un dispositif destiné à la chirurgie réfractive, lequel présente ce qui suit :
- une source de rayon laser (34),
- des moyens (36) pour former et guider le rayon laser émis par la source de rayon laser en rapport avec un oeil à traiter (10),
- une caméra (38) pour enregistrer l'iris et la pupille de l'oeil,
- un ordinateur (40) qui exécute un programme de commande pour commander lesdits moyens conformément à un profil d'ablation,
**caractérisé en ce que** le programme de commande :
- comporte une fonction acquise empiriquement pour l'oeil à traiter et concernant la position du sommet (A) de la cornée (12) de l'oeil (10) en fonction d'au moins une propriété de la pupille,
- enregistre à l'aide de ladite caméra la propriété momentanée de la pupille pendant l'intervention réfractive et calcule à partir de cette propriété la position du sommet (A) de la cornée au moyen de ladite fonction et
- centre le profil d'ablation sur la position du sommet (A) ainsi déterminée.

6. Procédé pour créer un programme de commande pour la chirurgie réfractive, à l'aide duquel un rayonnement laser est dirigé conformément à une succession spatio-temporelle prédéfinie sur ou dans un oeil à traiter, ladite succession spatio-temporelle prédéfinie étant déterminée par rapport à un endroit de l'oeil,
**caractérisé en ce que**
- la relation entre la position du sommet (A) et une propriété de la pupille qui peut changer au cours de l'intervention réfractive est déterminée empiriquement pour l'oeil à traiter et mémorisée avant la chirurgie réfractive.
